# EUROPEAN PATENT APPLICATION

(11) **EP 0 919 211 A2**
(43) Date of publication of application: **02.06.1999**
(21) Application number: 98309656.1
(22) Date of filing: 25.11.1998
(51) Int. Cl.: A61F 13/02

(54) **Wound dressing**

(30) Priority: 27.11.1997 GB 9725169
(71) Applicant: Beiersdorf Aktiengesellschaft, D-20253 Hamburg (DE)
(72) Inventor: Kenndoff, Jochen, Dr., 21629 Neu Wulmstorf (DE); Leutz, Reiner, 22465 Reinbek (DE); Fleck, Stephen George, Cambridge CB1 4PL (GB); Harman, Anthony David, Hertfordshire SG8 8DH (GB)
(74) Representative: Brunner, Michael John

(57) **Abstract**

A wound dressing (1) is formed from a release-coated, thermoformed plastics carrier film (2) which has a cavity (3) into which is dosed a measure of self-adhesive, hydrophillic polymer gel or gel foam dressing material (5). The cavity (3) in the carrier film has sloped or bevelled edges (4) to provide correspondingly tapered edges in the dressing material layer. An adhesive cover layer (6) is releasably supported on the carrier around the border (7) of the cavity (3) closing the cavity, and the dressing material is secured to it. A sealing film (8) is disposed over and releasably bonded to the adhesive cover layer (6).

## Description

The present invention relates to a wound dressing and particularly a dressing for application to ulcerated wounds or blisters.

International Patent Application PCT/EP93/02686, describes self-adhesive dressings based on a thin polyurethane rubber sheet laminated against or coated with a hydrocolloid gel or gel foam may be cut from a roll of prepared material and used as a wound dressing. The disadvantage of this method of producing dressings is that the sticky hydrocolloid material at the edges of the dressing is exposed to the environment, and may accidentally be wetted, or become adherent to clothes or bedsheets and lifted away from the wound site, as the edges of the dressing are square-cut and present an easy site for starting to peel the dressing from the skin.

Other prior art of background relevance includes EP-A-0264299 which describes a dressing with a permanent cover layer that has bevelled edges and a flat releasable cover layer. A similar construction is shown in WO-A-91/01706. WO-A-92/05755 discloses a wound dressing with a stepped cover layer.

According to the present invention there is provided a wound dressing comprising
a release-coated, thermoformed plastics carrier film in which is formed a cavity having sloped or bevelled edges;
a measured dose of self-adhesive, hydrophillic polymer gel or gel foam dressing material disposed within the cavity;
an adhesive cover layer releasably supported on the carrier around the border of the cavity closing the cavity, and to which the dressing material is secured; and
a sealing film disposed over and releasably bonded to the adhesive cover layer.

Preferably, the carrier comprises a pair of flange portions separable from the remainder of the carrier on opposite sides of the cavity whereby the cover layer, sealing film and dressing material can be removed together from the cavity by being peeled away from the cavity by hand using the separable flange portions as handles to prevent contamination and inactivation of the cover layer adhesive during application of the dressing to the skin.

The cover layer is advantageously very thin and flexible to allow conformation of the dressing to the curved surfaces of a wound site, especially at the borders of the dressing where unsupported cover layer areas are adhered directly to the skin to form a seal around the central pad that prevents accidental water ingress. The sealing layer is advantageously a stiff thin polymer film that can be releasably bonded to the cover layer to stiffen it and aid in handling and positioning of the dressing during application to the skin. On application to the skin, the bevelled portions of the central pad bend at very low force to allow secure adhesive contact with the skin. Once the middle of the dressing is in place on the skin, the flange portions are removable from the adhesive cover layer to allow full application of the adhesive cover layer to the wound. After application to the wound the sealing film can be removed from the cover layer by peeling it from the centre slit towards the edges at peel forces sufficiently low to avoid delamination of the dressing from the skin.

Preferably the cover layer comprises a polyurethane elastomer film coated on its underside with a pressure-sensitive adhesive material which temporarily seals with the release-coated surface of the carrier around the cavity and adheres to the dressing material within the cavity.

Advantageously the sealing film comprises a heat sealable plastics film adhered to the cover by the application of heat and pressure.

According to a second aspect of the present invention a wound dressing having a polyurethane cover layer additionally comprises a sealing layer in the form of a heat-sealable plastics film adhered to the surface of the covering layer.

Preferably, the sealing film is slit along a line across the dressing and edge portions of the sealing film on each side of the slit are not adhered to the covering layer. The un-adhered edge portions provide a simple means for enabling the sealing film to be peeled off the covering layer once the covering layer, carrying the wound dressing had been applied to a wound.

Alternatively, the sealing layer may have one or more pull tabs secured to the sealing film adjacent to an edge of the sealing film, whereby the sealing film can be peeled off the covering layer. Preferably there are two pull tabs, arranged on opposite sides of the dressing. The pull tab or tabs may be arranged to project beyond the edge of the dressing and may be connected to the sealing film over only part of its or their surface area. The pull tabs may assist in placing the wound dressing on the patient's skin.

The invention also includes a method of manufacturing a wound dressing, the method comprising
applying a release coating to a face of a plastics carrier;
thermoforming a cavity having sloped or bevelled edges in the release-coated plastics carrier, the cavity being formed in the face of the carrier to which the release coating is applied;
disposing a measured dose of self-adhesive, hydrophillic polymer gel or gel foam dressing material within the cavity;
releasably supporting a cover layer on the carrier around the border of the cavity to close the cavity, the cover layer having an adhesive on its face abutting the carrier and the dressing material;
adhering the dressing material (5) to the cover layer; and
providing a sealing film over and releasably bonded to the cover layer on its face opposite the dressing material.

The polymer gel is preferably disposed in the cavity by positioning the plastics carrier substantially horizontally with the cavity open upwards, and dosing the gel into the cavity to fill the cavity to provide edges tapering towards the cover layer. As the dressing and cover layer are `floppy' and stretchable, ie. of low modulus, and the tapered edges can thus bend easily to conform to a patient's skin, this avoids the need to manufacture the dressing by a more difficult method with the cover layer against the wall of the cavity, ie. with the taper towards the side of the dressing which is applied to the skin.

One example of a wound dressing and its method of manufacture will now be described with reference to the accompanying drawings in which:
Figure 1 is a plan view of a wound dressing;
Figure 2 is a cross-sectional view along the centre of Figure 1;
Figure 2A is a cross-sectional view similar to Figure 2, but of an alternative construction;

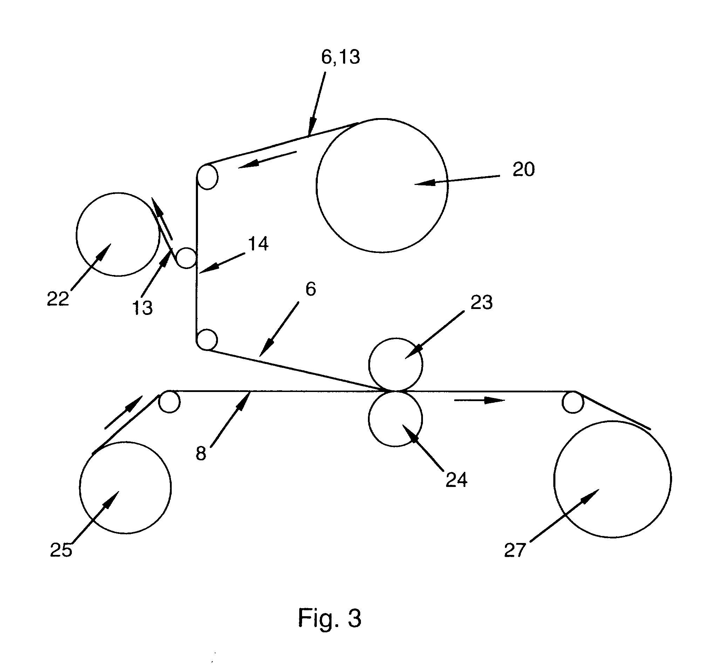

Figure 3 is a schematic view of a method of adhering a heat-sealable sealing film to a covering layer of the dressing;
Figure 4 is a schematic view of a prototype machine for assembling the wound dressing; and,
Figure 5 shows part of the process of Figure 4 in more detail.

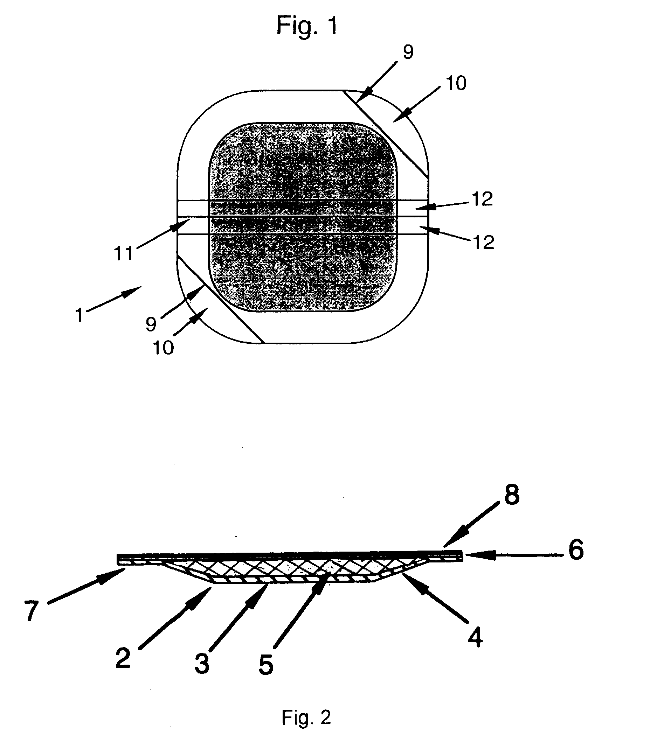

The wound dressing 1 shown in Figure 1 comprises a thermoformed (e.g. vacuum formed) carrier 2, the carrier comprising a suitable thermoforming plastics material such as polystyrene, polypropylene, polyethylene terepthalate or high density polyethylene, precoated on at least one of its surfaces with a silicone release material which prevents permanent adhesion of a cover layer 6 or the dressing material 5, and which maintains a functional release coating after thermoforming. Such plastics films are commercially available, and some grades of such material are currently approved for medical use. The carrier 2 is pre-formed with a dish or tray 3 having sloping edges 4. Lying within the dish or tray 3 is a polyurethane gel or gel foam mass 5 which is loaded with a super-absorbent hydrocolloid material to provide the wound dressing in use and which has tapering edges corresponding to the sloping sides of the dish or tray forming the cavity into which the dressing material is dosed. Provided over the wound dressing material is a polyurethane rubber film cover 6 which is coated on its underside with a pressure-sensitive adhesive film so that it is releasably secured to the thermo-formed carrier 2 around the dish or tray 3 at flanges 7. Over the cover layer 6 is provided a heat sealed polypropylene or polyester sealing layer 8 which protects the cover layer 6 until the dressing is actually used, and which, being relatively stiffer than the cover layer 6, acts as a temporary stiffening aid during the application of the dressing to the skin.

As best seen in Figure 1, the thermoformed carrier layer is partially cut or scored through its thickness along lines 9 to provide a pair "handles" 10 which are thus capable of being broken off the remainder of the carrier and by means of which the dressing can be removed from the tray 3 for use, the covering layer 6 being peeled back off the flanges 7, carrying the dressing material 5 with it. After application to the skin the handles can then likewise be peeled off the layer 6.

Centrally disposed across the dressing the sealing layer 8 is slit at 11, edge portions 12 on each side of the slit 11 being left un-adhered or un-bonded to the polyurethane rubber covering layer 6 so as to allow the sealing film 8 to be peeled back and removed from the cover layer 6 easily after application to the skin.

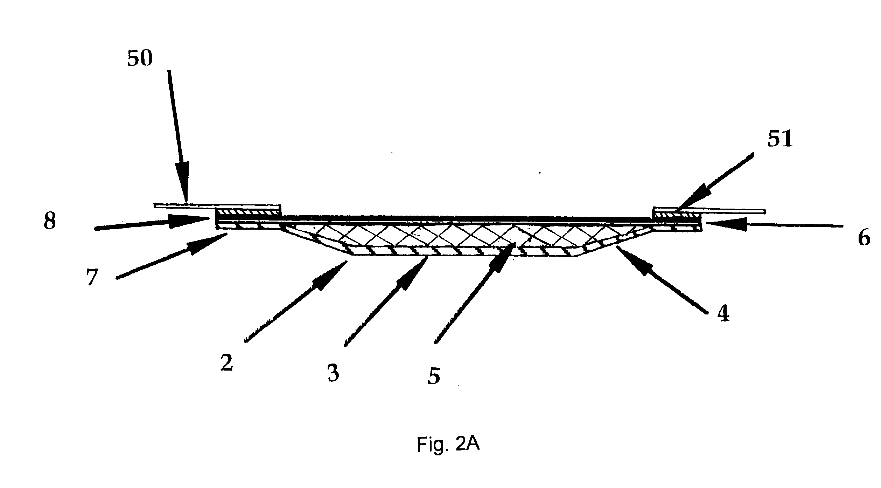

Alternatively, as shown in the modified example of Figure 2A, a pair of pull tabs 50 can be provided, one on each side of the dressing, to enable the sealing film 8 to be removed from the cover layer 6. These may also be used to position the dressing over a wound. The pull tabs may be adhesively attached to the sealing layer over part of their area only and project beyond the edge of the dressing.

In order to manufacture the carrier 2 with its associated dishes or trays 3, a conventional vacuum forming method is used which is not described herein detail as it is merely conventional roll-fed or sheet-fed of the type used for producing form, fill, seal food packaging trays with lids. It is important that the silicone release-coated side of carrier 2 is facing the dressing and that the release value is high enough to prevent delamination of the dressing from the cavity and flanges during manufacture, packaging, storage and use, but always allows the dressing to be peeled intact from the cavity by application of light hand forces.

In order to adhere the sealing film 8 to the covering layer 6, the polyurethane rubber material, which normally comprises a release-coated paper or plastics film on each side, is wound from a roll 20. The polyethylene release film 13 on one side is removed at a roller 21 and wound onto a further roller 22, while the polyurethane rubber film 6 is passed onwards between a pair of laminating rollers 23, 24. A heat sealable polypropylene or polyester sealing film 8 is fed from a roller 25 to the laminating rollers 23, 24 and there, by the application of suitable heat, pressure and contact time, is bonded or adhered to the polyurethane rubber film 6 to make a composite film that will delaminate by peeling under suitable application of light hand forces. In order to form the unbonded edge portions 12 shown in Figure 1, the laminating rollers are preferably formed with rebated portions so that the two layers are not under pressure at these portions as they pass between the rollers 23, 24 and thereafter the unbonded portion is subsequently slit by a knife 26. The combined polyurethane rubber and heat sealing film 6, 8 is wound up onto a further roller 27. On the side of the polyurethane rubber film opposite the release film 13 a further release layer 14 is provided which is not removed at this stage.

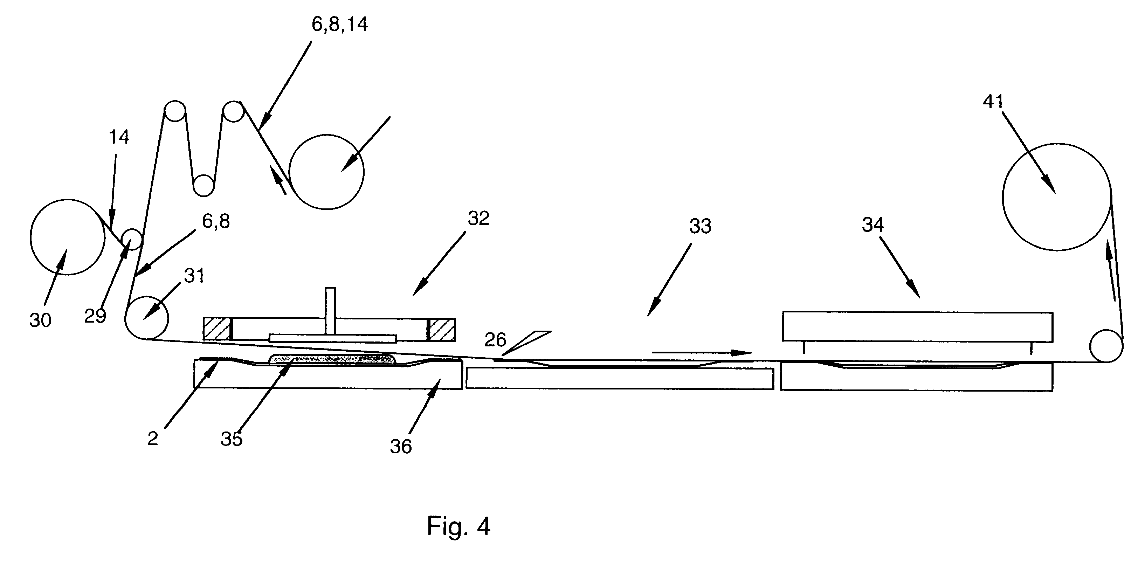

Subsequently, as seen in Figure 4, the roll of polyurethane rubber 6 with its release layer 14 and sealing layer 8 is unwound from a roller 28 and the release layer 14 peeled off the polyurethane rubber at the roller 29 and wound up onto a roller 30. The covering layer 6 and sealing layer 8 are then wound together around a roller 31 and through a final assembly which comprises a lidding station 32, a slitting station 33, and a punching station 34 as will now be described.

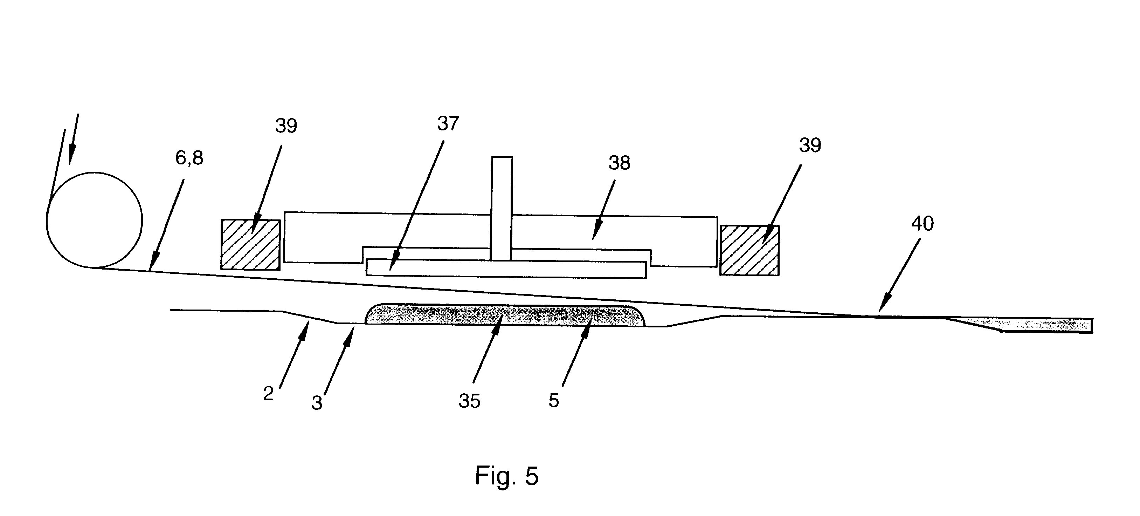

At the lidding station 32, as seen in more detail in Figure 5, the film layers 6, 8 are brought together with the carrier layer 2 into respective dishes or trays 3 of which the polyurethane/hydrocolloid dressing material 5 has been dosed as indicated at 35. A suitably shaped support 36 supports the underside of the dishes or trays and the layers 6, 8 are then pushed down onto the carrier layer 2 by a suitable contact plate 37. This step starts to apply the covering layer 6 to the dosed dressing material 35/5 and thereafter a surrounding platen 38 is lowered onto the film layers 6, 8 to start to spread the dose to fill the dish or tray 3. A clamp frame 39 is lowered onto the film layers 6, 8 to seal the covering layer 6 to the carrier layer 2 around the periphery of the dish or tray 3. Pockets of air that would otherwise be trapped inside the cavity following lidding are allowed to escape ahead of the spreading gel or gel foam dose through a series of small through-thickness holes or through a series of part-thickness depth channels which are preformed along the cavity rim of carrier layer 2, and especially at the corners of a square or rectangular shaped cavity. If a solid gel material is utilised for dressing material 5, then the dose may advantageously be spread by application of a vacuum to the cavity during the lidding operation. However, if a gel foam material is utilised, application of a vacuum during the lidding operation causes the cell structure of the foam to destabilise and become very non-uniform.

The platen assembly may be pivoted about the point of attachment of the films 6, 8 to the carrier layer 2 after the prior lidding step (point 40) to ensure parallel contact between the platen and the films 6, 8 and to further avoid air pockets within the dish or tray 3.

After the assembled dressing leaves the lidding station, still carried by the support 36, the sealing film 8 is slit by the knife 26 to provide the slit 11. At a punching station 34 the assembled dressing is cut from the remainder of the layer 2 into the shape shown in the plan view of Figure 1 and thereafter waste covering layer material 2 is wound up on a roller 41.

## Claims

1. A wound dressing (1) comprising
a release-coated, thermoformed plastics carrier film (2) in which is formed a cavity (3) ;
a measured dose of self-adhesive, hydrophillic polymer gel or gel foam dressing material (5) disposed within the cavity (3); and
an adhesive cover layer (6) releasably supported on the carrier around the border (7) of the cavity (3) closing the cavity, and to which the dressing material is secured; characterised by
a sealing film (8) disposed over and releasably bonded to the adhesive cover layer (6) ; and by
the cavity (3) in the carrier film having sloped or bevelled edges (4).

2. A wound dressing (1) according to claim 1, wherein the carrier (2) includes a flange portion (10) separable from the remainder of the carrier whereby the cover layer (6), sealing film (8) and dressing material (5) can be removed together from the cavity by being peeled away from the cavity by hand using the separable flange portion as a handle.

3. A wound dressing according to claim 2, including a pair of separable flange portions on opposite sides of the cavity (3).

4. A wound dressing (1) according to any claim 1 to 3, wherein the separable flange portions (10) are frangibly connected to the remainder of the carrier (6).

5. A wound dressing (1) according to any of claims 1 to 4, wherein the cover layer (6) is flexible to allow conformation of the dressing to the curved surfaces of a wound site.

6. A wound dressing (1) according to any of claims 1 to 5, wherein the sealing layer (8) is a relatively stiff, thin polymer film that is releasably bonded to the cover layer (6) to stiffen it and aid in handling and positioning of the dressing during application to the skin, and which may be removed from the cover layer thereafter.

7. A wound dressing (1) according to any of claims 1 to 6, wherein the cover layer (6) comprises a polyurethane elastomer film coated on its underside with a pressure-sensitive adhesive material which temporarily seals with the release-coated surface of the carrier (2) around the cavity and adheres to the dressing material within the cavity.

8. A wound dressing (1) according to any of claims 1 to 7, wherein the sealing film (8) comprises a heat-sealable plastics film adhered to the cover layer.

9. A wound dressing (1) having a polyurethane cover layer (6), characterised by a sealing layer (8) in the form of a heat-sealable plastics film adhered to a surface of the cover layer.

10. A wound dressing (1) according to any of claims 1 to 9, wherein the sealing film (8) is slit along a line (11) across the dressing and edge portions (12) of the sealing film on each side of the slit are not adhered to the covering layer (6).

11. A wound dressing (1) according to any of claims 1 to 9, further including a pull tab secured to the sealing film (8) adjacent to an edge of the sealing film, whereby the sealing film can be peeled off the covering layer (6).

12. A method of manufacturing a wound dressing (1), the method comprising
applying a release coating to a face of a plastics carrier (2) ;
thermoforming a cavity (3) having sloped or bevelled edges (4) in the release-coated plastics carrier (2), the cavity being formed in the face of the carrier to which the release coating is applied;
disposing a measured dose of self-adhesive, hydrophillic polymer gel or gel foam dressing material (5) within the cavity (3) ;
releasably supporting a cover layer (6) on the carrier around the border (7) of the cavity (3) to close the cavity, the cover layer having an adhesive on its face abutting the carrier and the dressing material;
adhering the dressing material (5) to the cover layer (6) ; and
providing a sealing film (8) over and releasably bonded to the cover layer (6) on its face opposite the dressing material (5) .

13. A method according to claim 10, wherein the polymer gel is disposed in the cavity by positioning the plastics carrier substantially horizontally with the cavity open upwards, and dosing the gel into the cavity to fill the cavity and hence provide edges tapering towards the cover layer.
